# EUROPEAN PATENT SPECIFICATION

(11) **EP 1 729 711 B1**
(45) Date of publication and mention of the grant of the patent: **22.04.2009**
(21) Application number: 05703180.9
(22) Date of filing: 04.02.2005
(51) Int. Cl.: A61H 1/02, A61H 3/00, A63B 23/035

(54) **REHABILITATION WITH MUSIC**
REHABILITATION MIT MUSIK
REEDUCATION FAISANT APPEL A LA MUSIQUE

(30) Priority: 05.02.2004 US 542022 P; 07.12.2004 US 633429 P
(43) Date of publication of application: 13.12.2006
(73) Proprietor: Motorika Ltd., Hamilton 12 Hamilton (BM)
(72) Inventor: EINAV, Omer, 42875 KFAR MONASH (IL); EINAV, Haim, 65149 Tel-Aviv (IL)
(74) Representative: van Westenbrugge, Andries
(86) International application number: PCT/IL2005/000137
(87) International publication number: WO 2005/086574

(56) References cited:
- EP-A- 0 304 538
- JP-A- 2003 164 544
- US-A- 5 397 865
- US-A- 5 846 086
- PATENT ABSTRACTS OF JAPAN vol. 014, no. 306 (C-0735), 3 July 1990 (1990-07-03) -& JP 02 102652 A (NIPPON AVIONICS CO LTD), 16 April 1990 (1990-04-16)
- PATENT ABSTRACTS OF JAPAN vol. 2002, no. 09, 4 September 2002 (2002-09-04) -& JP 2002 127058 A (SANYO ELECTRIC CO LTD), 8 May 2002 (2002-05-08)

## Description

### FIELD OF THE INVENTION

The present invention relates to a rehabilitation apparatus according to the preamble of claim 1.

### BACKGROUND OF THE INVENTION

A rehabilitation apparatus as defined in the preamble of claim 1 is known from JP-A-2003 164 544.

Rehabilitation of an injured person and/or training in the use of specific muscles requires tedious repetition of muscle movements. Generally such training is guided by a physical therapist or personal trainer teaching the person what moves to make and making sure that the person performs the moves.

People tend to get bored and tired of performing the exercises, especially if the motion is complicated, strenuous or causes pain.

Generally listening to music is considered enjoyable. Music typically transfers a rhythm to the listener and gives the user an urge to move. It is common practice to combine motion and music, for example as in dancing. Additionally, exercise and music are known to be combined, for example as in aerobics.

Music has also been used as background for physical therapy and rehabilitation.

### SUMMARY OF THE INVENTION

According to the invention, a rehabilitation apparatus as claimed in claim 1 is provided.

Some embodiments of the invention relate to devices that combines rehabilitation with music. In an exemplary embodiment of the invention, the device tracks a patient's motion and gives feedback to the patient (and/or a trainer or other person) using changes in music, for example indicating mistakes or generating comment such as an indication of speed of motion or smoothness of motion. In an exemplary embodiment of the invention, the device generates music to guide the person in performing rehabilitation. In one example, the device translates complex trajectories into music and music parameters that can be readily perceived. In another example, music is used to assist in synchronizing multiple body parts. In another example, music is used to give advance warning or a reminder of a change in motion, for example, a raising tempo indicating an upcoming change in direction of motion. In an exemplary embodiment of the invention, music is used as a guiding language for assisting in communication between an operator and a patient. In one example, an operator's motions are recorded spatially and using music and feedback to the operator on the quality of performance of such motions by a patient are provided using music.

In an exemplary embodiment of the invention, music is used for patients with cognitive impairment of the verbal system, who may not be able to understand, follow and/or generate verbal instructions or comment.

In some embodiments of the invention, music is used for physiological purposes, for example to motivate or calm down a patient, for example automatically in response to physiological sensing thereof. Optionally, music is used to convey general instructions, rather than instructions relating to a particular movement. In one example, music is used to indicate (e.g., a chime) that a series of exercises is reaching a last set.

In some embodiments of the invention music is used as an integral part of rehabilitation to add interest to the rehabilitation and/or fill in for times when a patient is too tired to perform an activity or during short rest periods. Optionally, music is used to provide motivation to a patient, e.g., a feeling that the exercises are actually having an effect on the real world.

In an exemplary embodiment of the invention, the use of music is used to convey a sense of time and/or timing to a rehabilitating person.

In some embodiments of the invention, a patient is required to move in a specific path or a desired location. Optionally alternatively or additionally, the patient is required to move at a specific rate or follow a particular tempo and/or rhythm.

According to the invention, at least one sensor adapted to sense a movement and generate a movement signal of the at least one motion support element is provided. Various sensors, such as position sensors, accelerometers or cameras can be used to track the motion of a patient (or other user).

In some embodiments of the invention, a part of the device is attached to the patient and moves with the patient. Optionally, the device guides motion by prohibiting or hindering certain motion, for example exerting force to cause the patient to move in a manner which follows a specific path and/or use the same tempo as the pre-selected music.

In an exemplary embodiment of the invention, a pre-selected music is played for the user responsive to the user motion. Optionally the music is played correctly if the user motion is performed correctly, for example correct timing and/or in the correct position. In some embodiments of the invention, the music is distorted, for example played offbeat or off tune, if the user motion is performed incorrectly. In some embodiments of the invention, additional sounds are added to signify errors in the motion, for example beeps or a verbal response.

In some embodiments of the invention, the device coordinates between motions of different parts of a patient's body, for example different channels indicating motions of different body parts.

In some embodiments of the invention, the device coordinates between motions of multiple users. In one example, the multiple users are multiple patients, for example, organized as a band, with each patient's rehabilitation activities sounding a different instrument. Differences in patient's abilities are optionally bridged by suitable devices, for example, one patient may be required to keep to a tempo within 10% and another be required to keep a motion to be circle-like, without restriction on speed or rhythm and with a precision of 50%. In both cases, if the requirements are met the devices will produce synchronized correct music. A potential advantage of such a setup is that a patient can feel that he is a productive part of a group. This may also provide motivation. In another example, the multiple users are a patient and an instructor. In another example, the multiple users are a patient and a layperson, such as a family member, which may assist in ensuring family support.

In an exemplary embodiment of the invention, games for rehabilitation are provided. In one example, the rehabilitation device is played as an instrument, for example, a patient plays drums by moving the device. Optionally, virtual reality techniques are used to provide feedback. In one example, a screen (or helmet) shows how the patient is hitting the drum. Optionally, the generated music is used as in karaoke where the patient needs to add his instrument to a pre-recorded band. Optionally, a pre-recorded band is provided via a connection to a communications network, such as the Internet.

In some embodiments of the invention, another visual feedback is used, for example showing a person doing the correct motions or giving encouragement using a visual device, for example a computer screen or a television.

There is thus provided in accordance with the invention an apparatus, comprising:
at least one sensor which generates an indication of a motion of a user in form of a movement signal of the motion support element;
a rhythmic audio generator; and
a controller configured to control said generator to modify the audio output thereof according to said indication.

In accordance with the invention, the rehabilitation apparatus also comprises:
the at least one motion support element adapted to support a motion of a part of a human; and the generator of rhythmic audio.
The controller is configured to correlate the audio output of said generator and said supported motion.

Optionally, said motion support element is adapted for attachment to a human.

Optionally, said motion support element is adapted for gripping by a human.

In an exemplary embodiment of the invention, said motion support element is adapted to assist a movement by a human. Optionally, said assisting comprises moving a portion of said human. Alternatively or additionally, said assisting comprises following a motion of said human while providing at least part of a motive force. Alternatively or additionally, said assisting comprises restricting a motion of said human.

In an exemplary embodiment of the invention, said motion support element is adapted to resist a movement by a human. Optionally, said resistance is not spatially uniform.

In an exemplary embodiment of the invention, said motion support element initiates said motion. Optionally, said motion support element moves said human. Alternatively or additionally, said motion support cues said human to start said motion.

In an exemplary embodiment of the invention, said controller generates said audio responsive to a correctness of said motion. Optionally, said controller modifies said audio during a motion according to a correctness of said motion. Said correctness may be judged against a stored plan. Said correctness may be judged against one or more criteria. Said controller may distort said audio according to a degree of error of said motion.

According to the invention, said controller generates said rhythmic audio to be timed to a desired movement by said human. Said audio can be generated before said movement. Said audio can be generated in time with said movement. Said audio can be generated during said movement including an anticipation of changes in said movement according to a plan stored in said controller.

In an exemplary embodiment of the invention, said controller is configured to generate a score according to a synchronization between movements to specific spatial locations and said audio. Alternatively or additionally, said controller is configured to mix a predetermined musical stream and audio generated according to said motion.

In an exemplary embodiment of the invention, said controller comprises a memory that links musical elements with motion elements. Optionally, said controller generates said audio from musical elements corresponding to different body parts. Alternatively or additionally, said controller generates said audio from musical elements corresponding to different motions.

In an exemplary embodiment of the invention, said controller generates said audio according to a difference between a desired motion and an actual motion.

In an exemplary embodiment of the invention, the apparatus comprises a sensor which generates an indication of said motion. Optionally, said controller generates a music stream according to said motion. Alternatively or additionally, said controller generates said stream as a set of instructions prior to detecting motion of said human. Alternatively or additionally, said controller generates series of musical notes and corresponding spatial motions.

In an exemplary embodiment of the invention, said controller has stored therein a plurality of trajectories of motion of said human.

In an exemplary embodiment of the invention, said controller has stored therein a rehabilitation program for said human.

In an exemplary embodiment of the invention, said audio comprises music.

In an exemplary embodiment of the invention, said audio generator is adapted to modify existing music.

In an exemplary embodiment of the invention, said controller is adapted to detect a physiological indicator of said human and generate music responsive thereto.

Additionally the controller may have stored therein rehabilitation information of a human,
wherein said controller is adapted to correlate said audio and said movement responsive to said rehabilitation information. Optionally, said apparatus is portable by an unassisted human. Optionally, said apparatus is wearable. Alternatively, said apparatus comprises a stable base and at least one moving extension.

A method of rehabilitation may comprise:
coupling a patient to a rehabilitation system;
performing a rehabilitation activity by said patient; and
automatically generating music correlated with said rehabilitation activity. Optionally, automatically generating comprises providing at least one cue to said patient. Alternatively or additionally, automatically generating comprises providing at least one musical instruction to said patient. Alternatively or additionally, automatically generating comprises providing feedback on a physical action using music. Alternatively or additionally, automatically generating comprises providing said music to other rehabilitated patients.

The method may comprise selecting music for a cognitively impaired patient. Automatically generating may comprise

Automatically generating may comprise generating music according to a correctness of motion.

Automatically generating may comprise generating music timed according to a desired motion.

Automatically generating may comprise requiring said patient to reach spatial locations according to a musical feature of said music.

Automatically generating may comprise generating a musical channel to overlay an existing musical channel according to a motion of said patient.

Automatically generating music may comprise generating music to synchronize motions of different points in a body of said patient.

Automatically generating music may comprises said patient bringing music to said system.

### BRIEF DESCRIPTION OF FIGURES

Particular exemplary embodiments of the invention will be described with reference to the following description of embodiments in conjunction with the figures, wherein identical structures, elements or parts which appear in more than one figure are generally labeled with a same or similar number in all the figures in which they appear, wherein:
Fig. 1A is a schematic illustration of a rehabilitation system 100 usable for integrating music and rehabilitation, in accordance with an exemplary embodiment of the invention;
Fig. 1B is a block diagram of a portion of a controller of Fig. 1A, in accordance with an exemplary embodiment of the invention;
Fig. 2 is a schematic illustration of a configuration including cameras and/or sensors for tracking user motion according to an exemplary embodiment of the invention;
Fig. 3 is a schematic illustration of a supportive input device according to an exemplary embodiment of the invention;
Fig. 4 is a flow diagram of a method of rehabilitating a user in accordance to an exemplary embodiment of the invention; and
Fig. 5 is a flow diagram of an alternative method of rehabilitating a user in accordance to an exemplary embodiment of the invention.

### DETAILED DESCRIPTION OF EMBODIMENTS

### Exemplary Device design

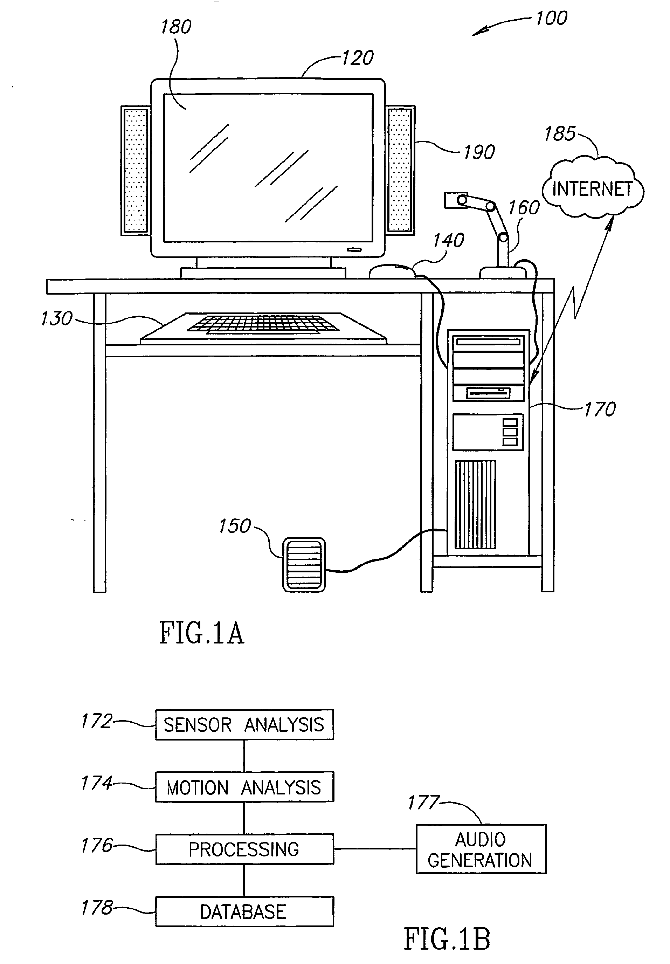

Fig. 1A is a schematic illustration of a rehabilitation system 100 usable for integrating music and rehabilitation, in accordance with an exemplary embodiment of the invention.

In an exemplary embodiment of the invention, rehabilitation system 100 comprises a controller, such as a personal computer 170 and an input device 160 for tracking user motion and inputting it into computer 170.

In an exemplary embodiment of the invention, input device 160 is optionally a standard input device of a computer, for example a joystick (not shown), a mouse 140, a keyboard 130, a trackball (not shown), a drawing tablet (not shown), a touch screen 180, a pedal 150 or any other standard input device. Alternatively or additionally, input device 160 is optionally a specially designed device, for example an articulated robot arm or other input devices as described in U.S. Pat. App. No. 60/542,022 and U.S. Pat. App. No. 60/633,442. In some embodiments of the invention, input device 160 is grasped by the user. Alternatively, input device 160 is attached to the user or supports a limb of the user depending on the type of motion performed with the input device as described below. Optionally, different input devices with different degrees of freedom are used depending on the muscles of the user that need to be trained and the level of control the user has of the muscles.

In some embodiments of the invention, multiple input devices 160 are provided, for example, for providing input from two or more limbs.

In an exemplary embodiment of the invention, computer 170 comprises an output device, for example display 120, which may be used for giving instructions and/or responses to the user.

In an exemplary embodiment of the invention, rehabilitation system 100 comprises speakers 190 used for playing music and/or other audio signals. Other audio sources may be used as well, for example a head set, for example a Bluetooth wireless head set.

Fig. 1B is a block diagram of a portion of controller 170, in accordance with an exemplary embodiment of the invention. This portion may be implemented, for example, using software, using hardware and/or using a combination thereof. In some embodiments, other organizational structures than the one shown in Fig. 1B are used, for example, an electronic circuit or a neural network.

A sensor analysis module 172 receives signals from sensors, for example position sensors and pressure sensors. Data from encoders of input device 160 may be converted, for example, into position information.

A motion analysis module 174 analyzes motion as reflected by the positions of body limbs, for example, determining smoothness of motion and/or spatial shape and/or speed of the motion.

A processor 176 then applies one of the methods described below, for example, for controlling an audio generator 177. Optionally, a database 178 of desired motions and/or music is used for applying such methods.

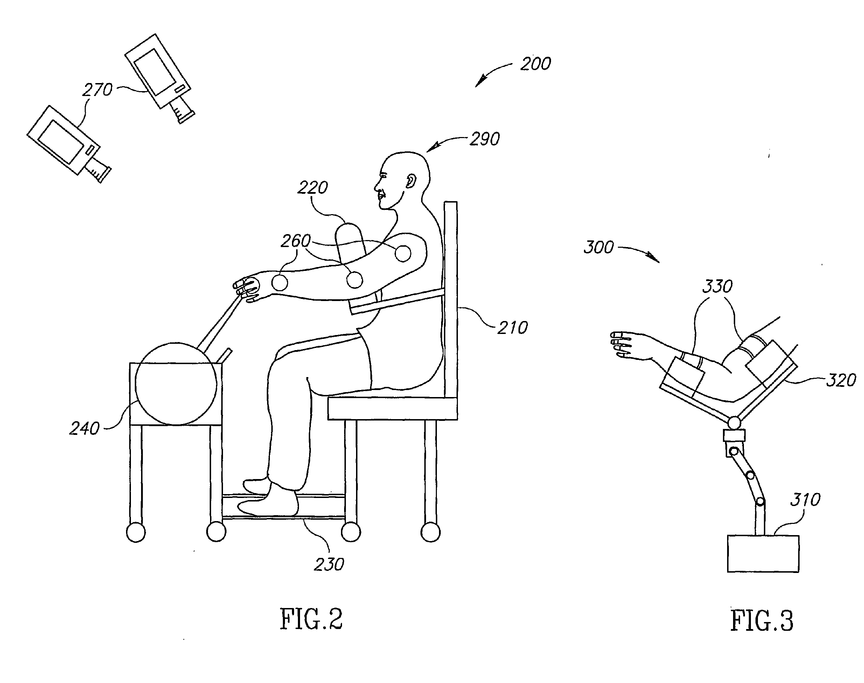

Fig. 2 is a schematic illustration of a rehabilitation configuration 200, which can serve as an input device 160 for rehabilitation system 100, according to an exemplary embodiment of the invention. In an exemplary embodiment of the invention, a disabled user 290, for example with a physical handicap, is seated in a chair 210. Optionally, user 290 is supported by a body rest 220 if user 290 needs support. In an exemplary embodiment of the invention, chair 210 is fixated with fixation bars 230 to a control device 240. Control device 240 is used by user 290 to train a damaged limb, for example a hand or a foot. In an exemplary embodiment of the invention, control device 240 is connected to computer 170 and transmits details of the motion performed by user 290. In some embodiments of the invention, control device 240 is controlled by computer 170 and trains user 290 by guiding the limb of user 290 to perform a specific motion. In some embodiments of the invention, the user's limb is attached to control device 240, for example using a strap, to prevent control device 240 from slipping away from the grasp of user 290. Control device 240 can be of various types, for example, it can be a ball with motors and/or a brake and an optional extending handle. Encoders may be provided on the ball and/or the handle, or on motors thereof. In another embodiment, control device 240 is an articulated robotic arm. Force sensors may be provided in the robotic arm. Pressure sensors may also be attached to parts of the body in contact with parts of system 100 or in contact with other items.

In some embodiments of the invention, rehabilitation device 200 comprises cameras or sensors 270 to detect the motion of user 290. Optionally, user 290 wears markers 260, for example fiduciary markers or transmitters to allow recording of the motion of a whole limb or other parts of the body of user 290. In some embodiments of the invention, the markers are passive markers that allow an external device to record motion based on them. Alternatively or additionally, the markers are active devices that sense and transmit a characteristic of the user motion, for example an accelerator meter that transmits acceleration, speed or an inclination sensor that senses and transmits the inclination of the sensor on the user relative to the perpendicular. Optionally, the markers include a feedback element that can provide feedback to patient, for example nudging the patient to move a limb in sync with the music. In an exemplary embodiment of the invention, cameras or sensors 270 transmit details of the motion of user 290 to computer 170 in order to record the motion and prepare feedback based on the motion. In some embodiments of the invention, cameras or sensors 270 track an absolute position of user 290 as a function of time. Alternatively or additionally, cameras or sensors 270 record a user's speed or acceleration.

Depending on the type of sensor used, one or more of the following informational items about the motion may be determined: speed, position, smoothness, acceleration, applied force and/or characteristic frequencies, for example of tremor. These characteristics can be, for example of particular parts of a body (e.g., a hand) or of multiple body parts taking part in a same motion (e.g., hand, wrist elbow and shoulder).

In some embodiments of the invention, sensors 270 comprise one or more physiological sensors, which record physiological parameters of a user, for example pulse rate, EMG, EEG, muscle tension and/or breathing rate. In an exemplary embodiment of the invention, rehabilitation system 100 responds immediately to these parameters, for example stop the exercise if the user is over stressed. Alternatively, the parameters may serve for general user progress reports. In an exemplary embodiment of the invention, the pulse rate is used as a feedback to vary music tempo, for example, to match to a speed, for providing feedback to the patient and/or to provide feedback to a caretaker. The actual signal may be indicative of limits rather than absolute values. In an exemplary embodiment of the invention, if stressing of the patient is determined, calling music is played, to reduce such tension. In other embodiments, for example, sensing EEG and EMG, the sensed signals relate directly to the rehabilitation, for example indicating correct (or incorrect) cognitive activation or muscle activation attempts. US provisional applications 60/604,615 and 60/566,078 describe examples of using EMG and EEG signals as part of rehabilitation.

While the present application focuses on audio presentation, in some embodiments of the invention, the audio presentation is supplemented by visual presentation. In one example, system 100 controls a DVD player or uses a TV set or a set-top box to display instructions and/or feedback. Optionally, visual rhythmic signals, for example, color waves are used, for example for deaf patients. The use of dual mode presentation may be helpful in some pathologies. It should be noted that control device 240 itself can serve as a feedback device, for example by vibrating or applying force, thus possibly providing three modes of feedback.

In an exemplary embodiment of the invention, musical feedback and/or instruction is used to assist in rehabilitating blind patients.

Fig. 3 is a schematic illustration of a supportive input device 300 according to an exemplary embodiment of the invention. In the embodiment of the invention shown in Fig. 3 a user's limb is strapped to plates 320 with straps 330. Optionally, the plates are mounted at the end of an articulated robot arm 310. In an exemplary embodiment of the invention, computer 170 is able to train a user's limb by controlling the movement of the user's limb with articulated robot arm 310 and/or moving plates 320.

### Types of Motion

The rehabilitation system 100 can be used in training users with different ability levels. As illustrated in Fig's 1-3 various devices can be used to affect different levels of control in training user 290 according to the ability of user 290. In some cases, user 290 has full control over the movement of the limb being trained. In other cases, user 290 has limited control or is only able to feel movement. In yet other cases, user 290 is unable to control or feel the limb.

In an exemplary embodiment of the invention, the ability of user 290 is taken into account in defining the type of motion user 290 is required to perform. In some cases, user 290 is required to repeat a specific motion or perform a specific motion, for example a circle or straight line. When the motion is being generated (by the user or the system) a musical or auditory cue and feedback is optionally given. The user rhythm is optionally measured and can be corrected or the user can be asked to correct it. Optionally, rehabilitation system 100 is programmed to be passive and allow any user motion. Alternatively or additionally, rehabilitation system 100 may offer resistance at various levels to the user's motion. In some embodiments of the invention the resistance may be uniform over a trajectory performed by the user. Alternatively or additionally, the resistance may act like a force field offering varying resistance at different points of the force field. In other cases rehabilitation system 100 is programmed to assist the user in moving his or her limb along a selected trajectory, for example once an initial motion in a correct direction was applied. In some cases system 100 corrects user mistakes, for example to within a predefined tolerance (e.g., if motion is in a generally correct direction, it is constrained to the exactly correct direction). In some cases rehabilitation system 100 may force the user to perform a specific motion. In one example, both the tempo of the music and the force applied by system 100 are directed towards causing a user to perform a motion at a desired velocity profile. In other cases, system 100 may apply force only if a user strays from a trajectory more than a given amount. In other cases, system 100 may remind the user of a required motion by providing a nudge. In some cases, system 100 serves to smooth user motion. Optionally, the intended motion is detected by measuring force applied by the user against control device 240.

In an exemplary embodiment of the invention, if input device 160 is assisting, resisting or forcing a user to perform motion rehabilitation system 100 measures the level of resistance of the user to the required motion, for example the measure of force the user exerts in different directions.

In an exemplary embodiment of the invention, the degree of resistance/assistance may be changed, for example, to determine a user's ability. Optionally, music used as a tempo or rhythm for rehabilitation is changed, for such testing.

Optionally, the quality of motion and/or a patient's ability are measured on a musical scale, for example, of accuracy, clarity, and/or pleasantness.

### Instruction

In an exemplary embodiment of the invention, a user is instructed visually, for example by the required motion being displayed on a display device, for example display 120 or a TV screen. Alternatively or additionally, the required motion is requested verbally, for example "move left hand up and down". In some embodiments of the invention the user can perform any type of motion as long as it correlates to attributes dictated by the music, for example tempo or path interval. Optionally the chosen user motion may be limited by input device 160, for example the user may move in any direction as long as he or she is grasping input device 160, which may apply a constraint on motion, for example a position and/or velocity constraint

In an exemplary embodiment of the invention, the user motion is displayed to the user as movement in a virtual world, for example a user's limb is shown as a controllable object on a display (e.g., goggles or a helmet) showing the virtual world. Optionally the virtual world may provide tasks which need to be performed by the user, for example touching certain objects or pushing objects with a minimum force.

A particular feature of some embodiments of the invention is the ability to bypass damaged neuronal pathways. In an exemplary embodiment of the invention, a user is instructed using music, rather than, or in addition to verbally and/or visually. In an exemplary embodiment of the invention, a stroke victim is taught a relationship between music and motion, for example by allowing motion and playing different music to match each motion. A more structured approach may be used as well, for example, moving a limb and playing suitable music at the same time. In an exemplary embodiment of the invention, system 100 coordinates the movement with the music, for example playing a predetermined music for each motion detected by it. Optionally, the effect of speed, smoothness, tremor and voice are also taught as affecting musical playback, for example, affecting one or more of tempo, one or more channels, amplitude, type of instrument and/or special effects (e.g., echo).

Once taught the rudiments of a musical language, physical rehabilitation can proceed in parallel with any speech rehabilitation.

In an exemplary embodiment of the invention, system 100 is designed to operate under voice control. In one example, the system is strained to recognize simple commands, like "stop", "slow down", "next" and "repeat". Optionally, the system recognizes musical commands, for example, whistling or rhythmic motion of control device 240. Alternatively or additionally, spatial gestures using device 240 are recognized. Optionally, musical feedback is provided to assist a user in determining that he made a correct gesture, while the gesture is being made and without necessarily requiring looking at the moving limb.

In an exemplary embodiment of the invention, instruction is provided by control device 240 moving, possibly while the user holds on to it. As will be described below, such a demonstration may also be accompanied by a musical playback.

### Using Music for Feedback

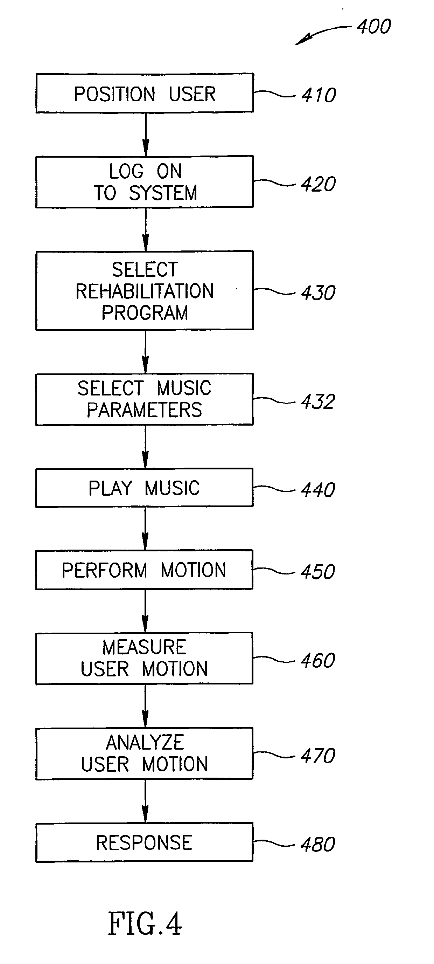

Fig. 4 is a flow diagram 400 of a method of rehabilitating a user using system 100, according to an exemplary embodiment of the invention. In an exemplary embodiment of the invention, a user is positioned (410) relative to rehabilitation system 100. In some cases, this may require strapping in. In some embodiments of the invention, the user optionally logs on (420) to computer 170 of rehabilitation system 100, so that system 100 can keep track of a user's progress, for example if the user is performing the required motion more accurately as a result of continuous practice. Alternatively rehabilitation system 100 is intended for a single user or a user supplies his or her personal data, for example on a diskette or other types of removable media. Optionally a user logs in using a smart card. Data regarding the user may be stored at a remote location. Optionally, the rehabilitation session is managed over a network, such as the Internet 185, for example a local network or a wide area network.

At 430, the user selects a rehabilitation program that is required for the user, for example the user may select between different stress levels or programs for dealing with different limbs. Optionally, the user selects between programs that perform different motion trajectories, for example moving in a curved path (e.g., a circle, an ellipse) or moving in a straight path (e.g., a line, a rectangle or forward and backward). As can be appreciated, in some cases, some of the above tasks of positioning, logging in and selecting an exercise may be performed by an operator, rather than by a patient. The selection of available exercises may be set in many ways, for example, automatically, according to a progress plan of the patient or manually, by an operator. Various rehabilitation parameters may be set, for example, limits, accuracy required, repetitions and/or any parameter known in the art.

In some embodiments of the invention, the rehabilitation program may be represented by a specific music, which the user is required to create by performing a motion matching the tempo of the selected music, for example moving at a specific rate and/or following a specific path. In some embodiments of the invention, the required motion is displayed to the user on display 120 (or control device 240 demonstrates it by moving itself) and the user is required to imitate the displayed motion. Music is optionally played during the demonstration and the same music is optionally generated if (and as) the user repeats the demonstrated motion.

At 432, one or more music parameters are optionally selected, for example, including one or more of music type (for example, quiet music or loud music), linkage of musical parameters to rehabilitation parameters (e.g., physiological or motion parameters).

In an exemplary embodiment of the invention, the music or class of music defines the stress level by defining a faster or slower tempo and/or a larger or smaller interval of motion, which is optionally related to attributes of the music.

In addition, music for setting an atmosphere may be selected.

Optionally, rehabilitation system 100 plays (440) the selected music. Optionally, the user brings music to be played on known media. The user is required to perform (450) a motion which is tracked by rehabilitation system 100 according to methods mentioned above. Optionally, the required motion and the music are selected and/or modified to match (and optionally provide guidance), for example as will be described below. In other embodiments, music is played only in response to a user motion, rather than during motion and/or being modified by the motion. In some cases, the user is not required to carry out a particular motion, rather any motion he carries out may have constraints set thereon, or free motion may be allowed.

In an exemplary embodiment of the invention, the music is synchronized to motion of control device 240, in that correct motion will create correct music. In another example, the motion of device 240 is made to match that of the music. In another example, device 240 assists, nudges and/or corrects a user motion so that it will match the music.

In an exemplary embodiment of the invention, rehabilitation system 100 measures and optionally records (460) the user motion as described above, for example by physical contact with an input to rehabilitation system 100 (e.g., a user grasping input device 160) or by non-physical contact with an input to rehabilitation system 100 (e.g., a camera recording the motion from a distance).

In an exemplary embodiment of the invention, rehabilitation system 100 analyzes (470) the recorded motion relative to the required motion to determine a level of correlation. In some embodiments of the invention, the analyzed results are logged for each user to determine their relative ability and/or if the user shows improvement.

In an exemplary embodiment of the invention, rehabilitation system 100 issues (480) a response to the user motion. In some embodiments of the invention, deviation from the required path is followed by an immediate response, for example distortion of the music being played and/or additional audio sounds such as beeps or other noises. Alternatively or additionally, rehabilitation system 100 may respond with a report after finishing an exercise session regarding the user's ability. In some embodiments of the invention, rehabilitation system 100 issues a report based on details from multiple uses of the device by a user. The report optionally deals with the improvement or lack of improvement of a user.

Immediate responses can be, for example discrete or continuous. In an example of a discrete response, a tone is played when a motion segment is correct or incorrect. In an example of a continuous response, a tempo relates to the speed of motion, like a metronome keeping the "beat" of the motion. In another example of a continuous response, the motion is construed to reflect a portion of music and system 100 plays a matching counter point. Possibly, two sets of feedback are thus available, one set in the music corresponding to the motion and one in the counterpoint music.

In an exemplary embodiment of the invention, a wave (or other format) file is pre-selected for each motion and/or for each position in space. If the position/motion is not reached exactly and/or on time, a distorted file will be played. Optionally, the distorted file includes a hint for one or more of amplitude of the error and direction of error.

It should be noted that not all parameters of the motion need to be responded to using music. For example, only one or more of the possible parameters are related to music. In one example, tempo is used for feedback on the speed of the motion, but the position is analyzed and presented using visual or kinesthetic means.

In an exemplary embodiment of the invention, the method of Fig. 4 is used for special rehabilitation exercises, for example arm rotations. Alternatively or additionally, this method is used for rehabilitation of daily activities. For example, different pieces of music may be associated with different activities, such as brushing teeth, tying shoelaces or walking. The music may serve as a reminder to the patient after rehabilitation is completed.

### Using music to guide

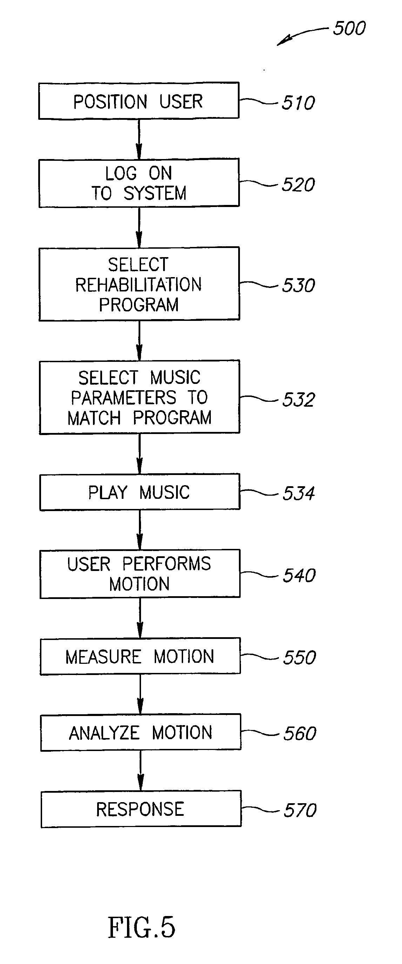

Fig. 5 is a flow diagram 500 of an alternative method of rehabilitating a user with rehabilitation system 100 in accordance with an exemplary embodiment of the invention. As in Fig. 4, a user is positioned (510) relative to rehabilitation system 100 and optionally logs on (520). After logging on, the user optionally selects (530) a specific rehabilitation program to perform.

At 532, musical parameters are selected to match the program. In an exemplary embodiment of the invention, the parameters are selected in a way which will guide a user, for example as described below. Selection may be, for example, automatic, from a table or manually.

At 534, the music is played and serves as a guide for a user performing a motion (540). In an exemplary embodiment of the invention, the music serves as a guide in one or more of the following manners:
(a) Tempo is used to tell user, for example, how fast to move, relative motion speeds (self calibration) and/or when to move. In particular, background music/tempo can be used to set the pace for the whole exercise or change during exercise as the user gets warmed up and/or slow down when the end is approaching. The user being warmed up may be detected, for example using physiological parameters, or based on time considerations. Also background music can be used to add interest during rest periods and/or during an exercise. Optionally, the music slows down when the patient stops and speeds up when motivation to try again is to be applied (e.g., decided manually or automatically).
(b) Feedback in general, for example as described with respect to Fig. 4, in that music is distorted by incorrect movement.
(c) Guidance on expected length of exercise and on time to do repetitions (for repetitive music).
(d) Training, in which music is used to train in doing exercises. For example, different musical phrases can be different motions which a patient learns. In another example, a set of morning exercises (tai chi) are taught using matching music.
(e) Synchronizing different body parts, for example, by a particular phase indicating combined motion or by first teaching each separate motion with its own music and then combining the motions and the music.
(f) Anticipation, for example by generating a tone or changing amplitude before a change of motion is required.
(g) Instruction, for example, particular musical phrases meaning "go to starting point", "faster" or "time to slow down".
(h) Warning. Music can be used to warn a user that he is approaching a point where he earlier felt pain or a point where a joint is unstable and care should be taken.
(i) Sequence of tones, with or without a given tempo, can be related to sequence of motion (e.g. to teach correct throwing of a ball, kicking, dancing).

The user's motion is measured (550) by rehabilitation system 100. Optionally, rehabilitation system 100 analyzes (560) the motion to match it to music and/or sounds based on the user's motion, for example the position of the user's limb, the rate of motion and/or acceleration. Exemplary feedback methods were described above. Optionally, in method 500, music and/or additional sounds are created (570) and optionally played for the user on the fly according to the user motion, for example music of a selected song is recreated with a tempo based on the rate of the user's motion. In another example, the movements emulate playing on a musical instrument.

In an exemplary embodiment of the invention, guidance and/or feedback are used to give a patient a sense of time and/or a sense of timing.

### Record and playback

In an exemplary embodiment of the invention, motions are programmed into system 100 by an instructor, in combination with music. For example, an instructor can show a correct motion and match it to music after the fact or perform to music. In an exemplary embodiment of the invention, position sensors (e.g., as shown in Fig. 2) are attached to the instructor and the position of various body parts recorded. In another example, the instructor moves the patient's limbs in a demonstration which is recorded, for later repetition by the patient. In an exemplary embodiment of the invention, the position sensors include electrical or vibrational stimulators.

In one example, the location change of each position sensor is recorded to a music-look up table of the following (or similar) form:

| Tune (name) | File name | Tempo | | |
|---|---|---|---|---|
| Yesterday | Yest.wav | xxx | | |
| Joint identifiers | Joint 1 | Joint 2 | Joint 3 | Joint x |
| | Left elbow | Right elbow | Left knee | Right knee |
| Musical Bar # | Joint 1 | Joint 2 | Joint 3 | Joint x |
| 1 | No motion | + | - | + |
| 2 | + | No | - | - |
| 3 | + | no | + | - |

| | | | | |
|---|---|---|---|---|
| Where "+" indicates motion away from the body. | | | | |

During playback, the patient plays the music (e.g., thru earphones). Controller 170 signals to each stimulator based on the above table and the patient responds accordingly. Position sensing and/or accelerometers may be used to determine if the motion is correct and optionally provide musical feedback

### Integrated motion controller

In an exemplary embodiment of the invention, a musical rehabilitation/exercise device is made portable. Such a device includes a controller and a plurality of stimulation patches. Headphones, optionally wireless are used to play music. The controller stimulates the patches according to the desired response by the user. Optionally, patches are provided in pairs or in greater numbers, for example for providing stimulation to a joint from multiple directions. Optionally, the patches include position sensors and/or accelerometers (or such are separately provided) for reporting to the controller the patient's response. In an exemplary embodiment of the invention, such a device is used for assistance in daily activities, with the stimulation and the music serving as reminders for the motions to be carried out. The stimulators and/or sensors may be provided in an article of clothing, for example a vest and/or be wireless. Optionally, the stimulation is electrical or vibrational. For deaf patients, vibration can be used to convey the rhythm and/or content of music.

### Body synchronization

As noted above, in an exemplary embodiment of the invention, musical means are used to help a patient synchronize motions between different body parts. In one example, a rehabilitation process is broken down into separate processes for each body part or for different motions of a same body part. Then a complex motion is guided by playing the music of multiple motions serially or in parallel, as appropriate. Delay in one channel may be used to indicate that its corresponding motion should be speeded up. In an exemplary embodiment of the invention, different body parts and/or motion are provided with different instrumental sounds.

In an alternative embodiment of the invention, music is used to indicate specifically the synchronization. For example, a piece of music is played correctly only if synchronization is correct, possibly using the same instruments for each body part as using during training. As noted above, the use of certain musical themes may be standardized so as to create a musical language. In one example, the left leg is always the sound of a flute and the right leg always an organ.

In an exemplary embodiment of the invention, rehabilitation system 100 and/or input device 160 may record motion from two limbs (e.g. arms or legs) simultaneously. In an exemplary embodiment of the invention, the user is required to perform an identical motion with both limbs. Alternatively or additionally, the user is required to perform mirrored motion, where the two limbs are required to perform mirrored motion. Optionally, the user is required to perform complementary motion or motion with a correlation between different limbs, for example throw an object from one hand to another.

### Instrument playing

In an exemplary embodiment of the invention, music is generated as if a user had one or more instruments. This may be part of a free-play session or it may be part of an exercise where particular motions are mandated. In an exemplary embodiment of the invention, the created music are selected according to the type of motion, for example a user can imitate playing a specific instrument such as a drum, guitar, violin, flute or any other instrument with a distinct playing pattern. In an exemplary embodiment of the invention, the various instruments are "positioned" in space, either as real instruments or as locations where they can be played by suitable motions. In an exemplary embodiment of the invention, a user plays such instruments in sequence, for example as set up by a therapist. Optionally, background music is provided to which such playing is to be synchronized. Optionally, the instruments are visible using virtual reality methods. Alternatively or additionally, the instruments include a light or other visual indication to remind a user when to "play" them. Optionally, an audible indication is used. Optionally, this allows different spatial locations to be mapped to musical sounds and/or instruments, as part of a rehabilitation language. In a session with pre-set motions, the musical generation may be made relative to the motions (e.g., to one or more of spatial, acceleration, .. force and/or velocity parameters). Alternatively or additionally, the generation is fixed in space. Optionally, as a user improves, the complexity of music created is increased. Alternatively or additionally, the degree of assistance is reduced. For example, a starting user may have a whole phrase generated just for making a motion, as time progresses, the user may get to a point where only a single note is generated and only if a correct motion is made. Exemplary intermediate levels are playing of a set of instruments, playing of a cord and triggering playback of channels.

In some embodiments of the invention, other parameters of the created music are selected by other aspects of the user motion, for example the height at which the motion is performed.

In an exemplary embodiment of the invention, control device 240 is used as a musical instrument. In one example, different locations, motions, speeds, and/or other musical parameters can make musical sounds. In an exemplary embodiment of the invention, the plane is mapped to various tones and the Z position is mapped to octave. Similarly, a mapping may be made in spherical coordinates. Optionally, the mapping matches a physiological motion. For example, the plane to which tones are matched is the surface of the edge of motion of a hand, with the octave being set based on the degree of bending of the elbow. This can give a user a feeling for range of motion and change in range of motion, especially if there is a problem with self-kinesthetic feedback.

In a tempo-teaching example, a user can reach for an instrument and a tone is generated only if the movement matched the tempo. Optionally, a time constraint is applied. Optionally, rehabilitation using music can be used to single out individual issues to be rehabilitated. For example, a user can perform an exercise whose purpose is to make a movement (e.g., any movement, a semi-correct movement, an exactly correct movement) at a given time. Conversely, rehabilitation can focus on the motion and provide music relating to the correctness of motion (e.g., music is fine as long as motion is within bounds) without training timing at the same time.

In a more complex example, a drum machine may be emulated, where different drums and other percussion instruments are positioned in different target volume of space. The applied force is easy to portray using amplitude.

Two drum sticks or a stick and a pedal (e.g., with position sensors) may be used for training two body parts simultaneously. System 100 can be used to demonstrate or instruct the user online on how to perform the music correctly. In general it should be noted that system 100 can stop a session and restart it or provide additional instruction or motivation, if results achieved by a user indicate it would be useful.

### Multi-user applications

In some embodiments of the invention, rehabilitation system 100 coordinates between multiple users, for example allowing competition between them or cooperative game playing. Optionally, the motion of different users can be assisted or hindered in order to reach a common level of complexity for users with varying ability. In some embodiments of the invention, a single rehabilitation system 100 can track multiple limbs and/or users, for example using a single camera and/or a single computer 170. Alternatively or additionally, multiple input devices 160 are used. In some embodiments of the invention, multiple computers 170 are used. Optionally, multiple users can practice or compete together over a communication line, for example multiple users each with a rehabilitation system 100 can participate in a common virtual reality environment over a LAN or the Internet 185.

A particular example of cooperation is in music making. Each user can have a different instrument. Optionally system 100 provides support (for example correction of movements) so that even severely impaired patients can take part. A potential advantage of such a setup is that impaired people can feel useful and take part in social activities, where a realistic level of participation is expected of them. Such a level is optionally determined by a therapist or automatically based on performance in exercises.

In an exemplary embodiment of the invention, music is used in a group therapy so that it is easy to portray to other users what a user did wrong. This is especially true if there are physical problems, such as the users being distributed and having a limited bandwidth and/or no cameras. Alternatively or additionally, music is used to help explain what motions were done.

In another example, a therapist or a computer acts as a conductor to guide a group session. In one example, a control device 240 is moved and music is sounded and the users repeat. This can be done in a band setting or not. As the users show their ability, the process may be made more complex and/or speeded up. As noted above, by providing support to weak users, such weak users can partake also if complexity increases by system 100 hiding such complexity and/or increasing a correction function of system 100.

In some cases, the users compete with each other, for example, to see who can create clearest music or while playing a game. Optionally, system 100 is used to even the odds between users of different abilities.

### Healthy-and-patient example

A particular example where music can be used to synchronize between multiple users is a therapist and a patient, where the music is used to synchronize their motions and/or allow the therapist to focus on a smaller number of mistakes in the motion, with other mistakes being "pointed out" by the music.

Another example is a home setting, where, for example, a layperson assists an elderly parent in rehabilitation. System 100 can compensate for lack of knowledge by providing music which guides both the healthy person and the parent-patient. The music serves to indicate what is correct and what should be corrected and the layperson can focus on caring and on helping such corrections to be carried out. It is believed that system 100 can thus reduce frustration and increase closeness in families with crippled members.

### Musical variations

As used herein, music is any type of audio with rhythm and can include, for example, chanting, and reading of prose, as well as periodic beeping. It is believed, however, the complex music and interesting music can have special benefit in providing multiple feedback possibilities, detailed feedback and/or interest. In particular, music can range between children's rhymes and complex orchestral pieces, for example, rock music, folksongs and pieces composed for a small number of instruments (e.g., 1, 2, 4 or 8).

In an exemplary embodiment of the invention, music is selected according to user preference. For example, a plurality of groups of music are available and a user selects one group. For example, groups can be "Rock", "Pop", Baroque" and "Chanting". Each group includes elements which can be used for the various uses described herein.

In an exemplary embodiment of the invention, music is selected for a particular rehabilitation task based on one or more of pace, beat, number of channels, regularity, existence, number and/or location of clearly identifiable turning points and liking and/or familiarity of the patient.

As noted above, different users have different abilities, so the actual parameters controlled by the user in a particular session may vary. For example, the parameters used may depend on one or more of: complexity of exercise, proficiency of user in music, rehabilitation stage of the user, ability of therapist, presence of participating group and/or mood.

In an exemplary embodiment of the invention, it is appreciated that a user is in a learning process when using music. Thus the complexity and/or speed of the music may increase over time. In an exemplary embodiment of the invention, the complexity, duration, type and/or difficulty of rehabilitation exercises increase over time, possibly at a different rate .. than music. In an exemplary embodiment of the invention, the number of instruments, tunes chords and/or cords used during rehabilitation is gradually increased. Optionally, additional musical variation (e.g., additional channels or instruments in an already existing musical piece) is introduced when a user shows advancing rehabilitation proficiency in one example, a vocal channel is added to provide feedback on smoothness of motion, once a user has shown proficiency in an exercise in which an instrument channel is linked to accuracy in controlling direction of motion.

In an exemplary embodiment of the invention, fixed parameters of music are mapped to rehabilitation parameters, for example, smoothness is linked to random variation in amplitude and accuracy of motion is linked to accuracy in reproduction of amplitude, frequency and/or tempo. As noted, different exercise may have different mappings. For example, in one exercise, any error smaller than 3 cm in position is ignored. In another, the effect of error on distortion of music is linear. In another, the effect is non-linear, for example exponential. The effect may be directional (e.g., increase in one direction and decrease in the other) or uni-directional, for example decrease in any direction away from correct direction. The term directions is used to represent any motion parameter, including one or more of position, velocity acceleration and force. Different effects may be used for patients with different disorders and/or depending on their stage or type of rehabilitation exercise used.

In some cases, music is used for psychological effect, in which case the effect of particular music on the patient is optionally gauged in a calibration session or over time. In one example, music is used to calm a patient who is over exerting. In some cases the effect is direct on the patient's emotions, in others the music serves as a signal to the patient (or therapist), or as a mirror of his emotional response.

In an exemplary embodiment of the invention, a user can provide his own music, for example a favorite music provided on CD. Such music can be used as background music or for a Karaoke game. In an exemplary embodiment of the invention, the music is analyzed (e.g., divided into channels) for use in rehabilitation. Optionally, the variations in the music which signify feedback and/or guidance are illustrated to the user prior to or during rehabilitation.

### Games

In an exemplary embodiment of the invention, music is used to provide fun during rehabilitation. In some embodiments, system 100 is used to support games, for example, single player games or multi-player games.

One example of a game is "Hit on Beat" in which a player gains points (or plays a sound) by reaching a desired spatial location and/or speed and/or acceleration and/or force at a time that coincides with a beat of music and/or before a musical timer runs out. Repeated "hits" may be supported.

Another example is "Musical Simon". Control device 240 makes a motion while patient 290 holds on and also sounds music at a same time. In a later level, possibly only the sound will be provided and the user will need to reconstruct the motion from memory. Only repeating correct motion (e.g., particular parameters such as position, velocity or others) and a correct sequence, will provide points. The motion may be constrained and/or assisted as described above. In a variant, the game is discrete and particular locations in space are associated with music. Repeating a set of musical tones requires a correct spatial motion sequence. Graphics are optionally used for visualization. In one example, a patient uses VR (or TV screen with graphical representation of motion), with different musical instruments each corresponding to a spatial target. The target size, shape and position can depend on the desired rehabilitation.

In a particular example not covered by claim 1, system 100 is used as a courser input device, with cursor positions mapped according to a particular rehabilitation exercise (e.g., drawing a circle, flexing an arm, etc.). The rules can be as follows: The game displays four different graphical signs. The player needs to replay the tracks by the moving the cursor to the graphical signs in the same order. For each iteration, the series of tracks grows one track longer, making it harder to replay the tracks in the correct order. For each good repeat the player will be awarded 1000 points. For out-of-tempo repeat he will be awarded 500 points. After the first wrong repeat the game will end and display a Game Over message and the score. The goal is to win as many points as possible. This is done by remembering as many musical notes as possible. The difficulty level can determine the music tempo.

In an exemplary embodiment of the invention, an interface module (software and/or hardware) is provided so that many cursor or joystick controlled games can be controlled using input device 160. The game may be on a same or on a different computer.

In general (for games and otherwise), exercises can be made more difficult by elongating the chain of causality between the instruction to the user and his performance. A shortest chain is the patient repeating a motion shown by a control 240. Intermediate is trying to reproduce a sound sequence (though sounds can be used as a hint to assist in a short-chain example). A still longer chain is a patient being taught spatial locations corresponding to sounds or instruments and expecting the patient to chain them together (e.g., plan motion trajectories) in response to hearing and anticipating a piece of music. This can train the speed and other characteristics of motion trajectory planning. In one example, some musical channels are provided and the patient provides the others. In another example, only words are shown and the patient needs to provide the music or only parts of it, for example a rhythm (absolute or relative values). As can be appreciated, music and motion control can be used to assist in cognitive rehabilitation, for example, for shape discrimination (e.g., recognizing shapes based on hand being moved along their outline and being shown), verbal processing (matching words to rhythm and/or motion), planning (e.g., of motion) and symbol sequencing (by doing ordered motion).

Another example of a game is Karaoke - a background tube is played while the user plays to the tune by one or more of: moving to a target instrument in space; moving between target instruments in space (a set of instruments is optionally placed by the therapist (or user or computer in space)); by just moving in the right direction; and/or by using multiple limb for more than one (VIRTUAL) instrument.

In an exemplary embodiment of the invention, games and/or music are used for rehabilitation of children. However, simpler music may need to be used for younger children. Additionally, music and common childhood games may be played with the assistance of rehabilitation system 100. Optionally, children group games are provided. Optionally, children are outfitted with sensors so that their motions can be tracked and responsive music generated

In an exemplary embodiment of the invention, music feedback and/or guidance is used to rehabilitate mentally damaged children, for example, children with Down's syndrome or children with Cerebral Palsy. Children with Cerebral Palsy often suffer from neglect of the paretic limb. In an exemplary embodiment of the invention, children with Cerebral Palsy are rehabilitated by providing a musical game that requires them to use the paretic limb to participate in the game.

Similarly, music feedback and/or guidance is optionally used to rehabilitate the aged as various body parts begin to lose function due to ailments, illness, physical trauma and general old age.

### Portability

In some embodiments of the invention, rehabilitation system 100 is constructed from elements which are lightweight and/or compact in order to allow portability of the device. Optionally, the elements of rehabilitation system 100 are easily assembled and calibrated so that a user is not restricted to a specific location. In some embodiments of the invention, rehabilitation system 100 is supplied as a kit with all necessary parts, for example folding tables and/or poles to position cameras, in order to limit dependency of rehabilitation system 100 on external elements of a specific location. Optionally, the system 100 is adapted to be used outdoors. Optionally, the system is used at home.

### Feedback

In an exemplary embodiment of the invention, the user receives immediate feedback, for example a distortion in the music being played or created responsive to the motion being performed. Optionally, delayed feedback is provided as well.

In some embodiments of the invention, rehabilitation system 100 supplies a user with a report regarding the user's performance relative to the requirements even for a single session. Optionally, rehabilitation system 100 supplies long term performance reports regarding a user's progression over a period of many sessions. In some embodiments of the invention, rehabilitation system 100 supplies reports regarding a user's performance relative to other users and/or an average user. In an exemplary embodiment of the invention, progress report are provided as musical files. In this way, a cognitively impaired and/or layman patient can be easily shown his progress.

In some embodiments of the invention, feedback reports can be accessed remotely by a user's therapist in order to monitor a user's progress, for example. Optionally, rehabilitation system 100 may send reports to a common database or directly to a therapist, for example by Email.

### General

The use of music for rehabilitation and exercise is not limited by the particular examples shown above. In particular, music can be used, for example for the fine motor control rehabilitation methods described in US provisional application 60/566,079. For fine motor control, music can be used to provide feedback for a task which the user is not supposed to be looking at, such as writing or eating and in which music is used to indicate if a motion is correctly executed.

Music can be used for neural rehabilitation. For example, US provisional application 60/604,615 uses neuronal sensing to determine when an action should be triggered. EEG signals can be used to modify music so a patient can recognize his neural activity.

Music can be used with EMG. For example, as described in US provisional application 60/566,078 musical signals can be used in addition to EMG or to prompt the patient that EMG is about to be or is being delivered. This may ':; increase sensitivity.

Music can be used with gait training, for example such as described in US provisional application 60/633,428. In one example, music is used to set the pace of walking or to synchronize the multiple body motions. In another example, music is used to help a patient tell which one of the body motions (e.g., hips, legs, feet, shoulders) and on which body side (e.g., using stereo) is out of synch.

Music can be used for balance training, for example such as described in US provisional application 601633,442. In one example, music is used to indicate balance between body sides. A channel which is too loud may be used to indicate a body portion applying too much force. Silence may be used to indicate balance, while a wobble will generate a cyclical tune and as balance is lost an alarm may increase in amplitude.

## Claims

1. Rehabilitation apparatus (100, 200), comprising:
at least one motion support element (160, 240, 320) adapted to support a motion of a part of a human (290);
at least one sensor (260, 270) adapted to sense a movement and generate a movement signal of said at least one motion support element (160, 240, 320); and
a generator of audio (190);
a controller (170) in communication with said generator (190) and said at least one sensor (260, 270), said controller adapted to:
control said generator of audio (190) to generate rhythmic audio timed to a stored desired movement of said human (290);
receive said sensed movement signal from said at least one sensor (260, 270); **characterized in that** the controller is adapted to
modify said generator provided rhythmic audio in accordance with said sensed movement signal.

2. Apparatus according to claim 1, wherein said motion support element (160, 240, 320) is adapted for attachment to a human.

3. Apparatus according to claim 1, wherein said motion support element (160, 240, 320) is adapted for gripping by a human.

4. Apparatus according to any of claims 1-3, wherein said motion support element (160, 240, 320) is adapted to assist a movement by a human.

5. Apparatus according to claim 4, wherein said assisting comprises moving a portion of said human.

6. Apparatus according to claim 4 or claim 5, wherein said assisting comprises following a motion of said human while providing at least part of a motive force.

7. Apparatus according to any of claims 4-6, wherein said assisting comprises restricting a motion of said human.

8. Apparatus according to any of claims 1-7, wherein said motion support element (160, 240, 320) is adapted to resist a movement by a human.

9. Apparatus according to claim 8, wherein said resistance is not spatially uniform.

10. Apparatus according to any of claims 1-9, wherein said motion support element (160, 240, 320) initiates said motion.

11. Apparatus according to claim 10, wherein said motion support element (160, 240, 320) moves said human (290).

12. Apparatus according to claim 10 or claim 11, wherein said motion support element cues said human (290) to start said motion.

13. Apparatus according to any of claims 1-12, wherein said controller is configured to generate said audio responsive to a correctness of said sensed movement in comparison with said stored desired movement.

14. Apparatus according to claim 13, wherein said controller is configured to modify said audio during a motion according to a correctness of said sensed movement.

15. Apparatus according to claim 13 or claim 14, wherein said controller is configured to distort said audio according to a degree of error of said sensed movement in comparison with said stored desired movement.

16. Apparatus according to claim 1, wherein said audio is generated in time with said movement.

17. Apparatus according to claim 1, wherein at least one plan is stored in said controller which said controller is configured to use for preceding a required change in said desired movement with a change in said audio.

18. Apparatus according to claim 16 or claim 17, wherein said controller is configured to generate a score according to a synchronization between sensed movements to specific spatial locations and said audio.

19. Apparatus according to any of claims 16-18, wherein said controller is configured to mix a predetermined musical stream and an audio generated according to said sensed movement.

20. Apparatus according to any of claims 16-19, wherein said controller comprises a memory storing links between musical elements and motion elements.

21. Apparatus according to claim 20, wherein said memory stores musical elements in correspondence with body parts and said controller is configured to generate said audio from musical elements corresponding to different body parts.

22. Apparatus according to claim 20, wherein said controller is configured to generate said audio from musical elements linked with different motion elements.

23. Apparatus according to any of claims 1-22, wherein said controller is configured to generate said audio according to a difference between a stored desired movement and a sensed movement.

24. Apparatus according to any of claims 1-23, wherein said controller is configured to analyze said movement signal to generate a music stream according to said movement signal.

25. Apparatus according to any of claims 1-24, wherein said controller is configured to generate said audio prior to detecting motion of said human (290).

26. Apparatus according to any of claims 1-25, wherein said controller is in communication with said motion support element (160, 240, 320) and is configured to control the audio generator (190) to generate series of musical notes and to control the (160, 240, 320) support element to support corresponding spatial motions.

27. Apparatus according to any of claims 1-26, wherein said controller has stored therein a plurality of trajectories of motion of said human (290).

28. Apparatus according to any of claims 1-27, wherein said controller comprises a memory storing therein a rehabilitation program for said human (290).

29. Apparatus according to any of claims 1-28, wherein said audio comprises music.

30. Apparatus according to any of claims 1-29, wherein said audio generator (190) is adapted to modify existing music.

31. Apparatus according to claim 1, comprising a detector adapted to detect a physiological indicator of said human (290) and in communication with said controller and wherein said controller is configured to control the generator (190) to generate music responsive to said detected physiological indicator.

32. Apparatus according to any of claims 1-31, wherein said apparatus is portable by an unassisted human.

33. Apparatus according to any of claims 1-32, wherein said apparatus is wearable.

34. Apparatus according to any of claims 1-33, wherein said apparatus comprises a stable base and at least one moving extension.

## Patentansprüche

1. Rehabilitationsvorrichtung (100, 200), umfassend:
zumindest ein Bewegungsunterstützungselement (160, 240, 320), das eingerichtet ist, eine Bewegung eines Teils eines Menschen (290) zu unterstützen;
zumindest einen Sensor (260, 270), der eingerichtet ist, eine Bewegung abzufühlen und ein Bewegungssignal zu erzeugen, das sich auf das zumindest eine Bewegungsunterstützungselement (160, 240, 320) bezieht; und
einen Schallerzeuger (190),
eine Steuerung (170), die in Verbindung mit dem Generator (90) und mit dem zumindest einen Sensor (260, 270) ist, wobei die Steuerung eingerichtet ist, um:
den Schallerzeuger (190) zu steuern, rhythmischen Schall zu erzeugen, der gemäß einer gesteuerten gewünschten Bewegung des Menschen (290) im zeitlichen Verlauf bestimmt ist; und
das geführte Bewegungssignal von dem zumindest einen Sensor (260, 270) zu empfangen; **dadurch gekennzeichnet, dass** die Steuerung eingerichtet ist, den vom Generator vorgesehenen rhythmischen Schall gemäß dem abgefühlten Bewegungssignal zu modifizieren.

2. Vorrichtung nach Anspruch 1, wobei das Bewegungsunterstützungselement (160, 240, 320) zur Befestigung an einem Menschen eingerichtet ist.

3. Vorrichtung nach Anspruch 1, wobei das Bewegungsunterstützungselement (160, 240, 320) zum Greifen durch einen Menschen eingerichtet ist.

4. Vorrichtung nach einem der Ansprüche 1-3, wobei das Bewegungsunterstützungselement (160, 240, 320) eingerichtet ist, bei einer von einem Menschen ausgeführte Bewegung mitzuwirken.

5. Vorrichtung nach Anspruch 4, wobei das Mitwirken umfasst: Bewegen eines Teils eines Menschen.

6. Vorrichtung nach Anspruch 4 oder Anspruch 5, wobei das Mitwirken umfasst: Folgen einer Bewegung des Menschen, während zumindest ein Teil einer Bewegungskraft vorgesehen wird.

7. Vorrichtung nach einem der Ansprüche 4-6, wobei das Mitwirken umfasst: Einschränken einer Bewegung des Menschen.

8. Vorrichtung nach einem der Ansprüche 1-7, wobei das Bewegungsunterstützungselement (160, 240, 320) eingerichtet ist, einer Bewegung eines Menschen zu widerstehen.

9. Vorrichtung nach Anspruch 8, wobei der Widerstand nicht räumlich gleichförmig ist.

10. Vorrichtung nach einem der Ansprüche 1-9, wobei das Bewegungsunterstützungselement (160, 240, 320) die Bewegung initiiert.

11. Vorrichtung nach Anspruch 10, wobei das Bewegungsunterstützungselement (160, 240, 320) den Menschen (290) bewegt.

12. Vorrichtung nach einem der Ansprüche 10 oder 11, wobei das Bewegungsunterstützungselement den Menschen (290) anweist, die Bewegung zu starten.

13. Vorrichtung nach einem der Ansprüche 1-12, wobei die Steuerung konfiguriert ist, den Schall in Reaktion auf die Korrektheit der abgefühlten Bewegung im Vergleich mit der gespeicherten gewünschten Bewegung zu erzeugen.

14. Vorrichtung nach Anspruch 13, wobei die Steuerung eingerichtet ist, den Schall während einer Bewegung gemäß einer Korrektheit der abgefühlten Bewegung zu modifizieren.

15. Vorrichtung nach Anspruch 13 oder Anspruch 14, wobei die Steuerung eingerichtet ist, den Schall gemäß einem Fehlermaß der abgefühlten Bewegung im Vergleich mit der gespeicherten gewünschten Bewegung zu verzerren.

16. Vorrichtung nach Anspruch 1, wobei der Schall in zeitlicher Übereinstimmung mit der Bewegung erzeugt wird.

17. Vorrichtung nach Anspruch 1, wobei zumindest ein Plan in der Steuerung gespeichert ist, wobei die Steuerung eingerichtet ist, zur Vorwegnahme einer erforderlichen Veränderung der gewünschten Bewegung durch eine Änderung in dem Schall verwendet zu werden.

18. Vorrichtung nach Anspruch 16 oder Anspruch 17, wobei die Steuerung konfiguriert ist, eine Punktzahl gemäß einer Synchronität zwischen abgefühlten Bewegungen gemäß spezifischen räumlichen Orten und dem Schall zu erzeugen.

19. Vorrichtung nach einem der Ansprüche 16-18, wobei die Steuerung konfiguriert ist, einen vorbestimmten Musikstrom und einen Schall, der gemäß der gewünschten Bewegung erzeugt wird, zu mischen.

20. Vorrichtung nach einem der Ansprüche 16-19, wobei die Steuerung einen Speicher umfasst, der Verknüpfungen zwischen Musikelementen und Bewegungselementen speichert.

21. Vorrichtung gemäß Anspruch 20, wobei der Speicher Musikelemente in Entsprechung mit Körperteilen speichert, und die Steuerung konfiguriert ist, den Schall aus Musikelementen, die verschiedenen Körperteilen entsprechen, zu erzeugen.

22. Vorrichtung nach Anspruch 20, wobei die Steuerung konfiguriert ist, das Audiosignal aus Musikelementen zu erzeugen, die mit verschiedenen Bewegungselementen verknüpft sind.

23. Vorrichtung nach einem der Ansprüche 1-22, wobei die Steuerung eingerichtet ist, den Schall gemäß einem Unterschied zwischen einer gespeicherten gewünschten Bewegung und einer abgefühlten Bewegung zu erzeugen.

24. Vorrichtung nach einem der Ansprüche 1-23, wobei die Steuerung konfiguriert ist, das Bewegungssignal zu analysieren, um einen Musikstrom gemäß dem Bewegungssignal zu erzeugen.

25. Vorrichtung nach einem der Ansprüche 1-24, wobei die Steuerung eingerichtet ist, den Schall vor der Erfassung der Bewegung des Menschen (290) zu erzeugen.

26. Vorrichtung nach einem der Ansprüche 1-25, wobei die Steuerung in Verbindung mit dem Bewegungsunterstützungselement (160, 240, 320) ist, und eingerichtet ist, den Schallgenerator (190) zu steuern, um eine Folge von Musiknoten zu erzeugen und um das Unterstützungselement (160, 240, 320) zur Unterstützung entsprechender räumlicher Bewegungen zu steuern.

27. Vorrichtung nach einem der Ansprüche 1-26, wobei in der Steuerung eine Vielzahl von Bewegungsortskurven des Menschen (290) gespeichert ist.

28. Vorrichtung nach einem der Ansprüche 1-27, wobei die Steuerung einen Speicher umfasst, in dem ein Rehabilitationsprogramm für den Menschen (290) gespeichert ist.

29. Vorrichtung nach einem der Ansprüche 1-28, wobei der Schall Musik umfasst.

30. Vorrichtung nach einem der Ansprüche 1-29, wobei der Schallgenerator (190) eingerichtet ist, bestehende Musik zu modifizieren.

31. Vorrichtung nach Anspruch 1, umfassend einen Detektor, der eingerichtet ist, einen physiologischen Indikator des Menschen (290) zu erfassen, und in Verbindung mit der Steuerung ist, und wobei die Steuerung konfiguriert ist, den Generator (190) zu steuern, um Musik in Antwort auf den erfassten physiologischen Indikator zu erzeugen.

32. Vorrichtung nach einem der Ansprüche 1-31, wobei die Vorrichtung für einen Menschen portabel ist, der nicht unterstützt wird.

33. Vorrichtung nach einem der Ansprüche 1-32, wobei die Vorrichtung tragbar ist.

34. Vorrichtung nach einem der Ansprüche 1-33, wobei die Vorrichtung eine stabile Basis umfasst und zumindest eine bewegliche Erweiterung.

## Revendications

1. Dispositif de rééducation (100, 200) comprenant :
au moins un élément de support de mouvement (160, 240, 320) adapté pour supporter un mouvement d'une partie d'une personne (290) ;
au moins un capteur (260, 270) adapté pour détecter un mouvement et générer un signal de mouvement dudit au moins un élément de support de mouvement (160, 240, 320) et
un générateur de signal audio (190) ;
un dispositif de commande (170) en communication avec ledit générateur (190) et ledit au moins un capteur (260, 270), ledit dispositif de commande étant adapté pour :
commander ledit générateur de signal audio (190) pour générer un signal audio rythmique synchronisé avec un mouvement désiré mémorisé de ladite personne (290) ;
recevoir ledit signal de mouvement détecté provenant dudit au moins un capteur (260, 270) ;
**caractérisé en ce que** ledit dispositif de commande est adapté pour modifier ledit signal audio rythmique délivré par le générateur en fonction dudit signal de mouvement détecté.

2. Dispositif selon la revendication 1, dans lequel ledit élément de support de mouvement (160, 240, 320) est adapté pour la fixation sur une personne.

3. Dispositif selon la revendication 1, dans lequel ledit élément de support de mouvement (160, 240, 320) est adapté pour la préhension par une personne.

4. Dispositif selon l'une quelconque des revendications 1 à 3, dans lequel ledit élément de support de mouvement (160, 240, 320) est adapté pour assister un mouvement par une personne.

5. Dispositif selon la revendication 4, dans lequel ladite assistance comprend le déplacement d'une partie de ladite personne.

6. Dispositif selon la revendication 4 ou 5, dans lequel ladite assistance comprend le suivi d'un mouvement de ladite personne tout en fournissant au moins une partie d'une force motrice.

7. Dispositif selon l'une quelconque des revendications 4 à 6, dans lequel ladite assistance comprend la restriction d'un mouvement de ladite personne.

8. Dispositif selon l'une quelconque des revendications 1 à 7, dans lequel ledit élément de support de mouvement (160, 240, 320) est adapté pour résister à un mouvement par une personne.

9. Dispositif selon la revendication 8, dans lequel ladite résistance n'est pas uniforme spatialement.

10. Dispositif selon l'une quelconque des revendications 1 à 9, dans lequel ledit élément de support de mouvement (160, 240, 320) démarre ledit mouvement.

11. Dispositif selon la revendication 10, dans lequel ledit élément de support de mouvement (160, 240, 320) déplace ladite personne (290).

12. Dispositif selon la revendication 10 ou 11, dans lequel ledit élément de support de mouvement invite ladite personne (290) à commencer ledit mouvement.

13. Dispositif selon l'une quelconque des revendications 1 à 12, dans lequel ledit dispositif de commande est configuré pour générer ledit signal audio en réponse à une justesse dudit mouvement détecté en comparaison avec ledit mouvement désiré mémorisé.

14. Dispositif selon la revendication 13, dans lequel ledit dispositif de commande est configuré pour modifier ledit signal audio durant un mouvement en fonction d'une justesse dudit mouvement détecté.

15. Dispositif selon la revendication 13 ou 14, dans lequel ledit dispositif de commande déforme ledit signal audio en fonction d'un degré d'erreur dudit mouvement détecté en comparaison avec ledit mouvement désiré mémorisé.

16. Dispositif selon la revendication 1, dans lequel ledit signal audio est généré en synchronisation avec ledit mouvement.

17. Dispositif selon la revendication 1, dans lequel au moins un programme est mémorisé dans ledit dispositif de commande, ledit dispositif de commande étant configuré pour l'utiliser pour précéder un changement nécessaire dudit mouvement désiré avec une variation dudit signal audio.

18. Dispositif selon la revendication 16 ou 17, dans lequel ledit dispositif de commande est configuré pour générer un score en fonction d'une synchronisation entre des mouvements détectés pour des positions spatiales spécifiques et ledit signal audio.

19. Dispositif selon l'une quelconque des revendications 16 à 18, dans lequel ledit dispositif de commande est configuré pour mixer un flux musical prédéterminé et un signal audio généré en fonction dudit mouvement détecté.

20. Dispositif selon l'une quelconque des revendications 16 à 19, dans lequel ledit dispositif de commande comprend une mémoire stockant des liens entre éléments musicaux et éléments de mouvement.

21. Dispositif selon la revendication 20, dans lequel ladite mémoire mémorise des éléments musicaux en correspondance avec des parties du corps et ledit dispositif de commande est configuré pour générer ledit signal audio à partir d'éléments musicaux correspondant à différentes parties du corps.

22. Dispositif selon la revendication 20, dans lequel ledit dispositif de commande est configuré pour générer ledit signal audio à partir d'éléments musicaux liés à des éléments de mouvement différents.

23. Dispositif selon l'une quelconque des revendications 1 à 22, dans lequel ledit dispositif de commande est configuré pour générer ledit signal audio en fonction d'une différence entre un mouvement désiré mémorisé et un mouvement détecté.

24. Dispositif selon l'une quelconque des revendications 1 à 23, dans lequel ledit dispositif de commande est configuré pour analyser ledit signal de mouvement pour générer un flux musical en fonction dudit signal de mouvement.

25. Dispositif selon l'une quelconque des revendications 1 à 24, dans lequel ledit dispositif de commande est configuré pour générer ledit signal audio avant de détecter le mouvement de ladite personne (290).

26. Dispositif selon l'une quelconque des revendications 1 à 25, dans lequel ledit dispositif de commande est en communication avec ledit élément de support de mouvement (160, 240, 320) et est configuré pour commander le générateur de signal audio (190) pour générer des séries de notes de musique et pour commander l'élément de support (160, 240, 320) pour supporter des mouvements spatiaux correspondants.

27. Dispositif selon l'une quelconque des revendications 1 à 26, dans lequel une pluralité de trajectoires de mouvement de ladite personne (290) est mémorisée dans ledit dispositif de commande.

28. Dispositif selon l'une quelconque des revendications 1 à 27, dans lequel ledit dispositif de commande comprend une mémoire qui stocke un programme de rééducation pour ladite personne (290).

29. Dispositif selon l'une quelconque des revendications 1 à 28, dans lequel ledit signal audio comprend une musique.

30. Dispositif selon l'une quelconque des revendications 1 à 29, dans lequel ledit générateur de signal audio (190) est adapté pour modifier une musique existante.

31. Dispositif selon la revendication 1, comprenant un détecteur adapté pour détecter un indicateur physiologique de ladite personne (290) et en communication avec ledit dispositif de commande et dans lequel ledit dispositif de commande est configuré pour commander le générateur (190) pour générer une musique en fonction dudit indicateur physiologique détecté.

32. Dispositif selon l'une quelconque des revendications 1 à 31, dans lequel ledit dispositif est portable par une personne non assistée.

33. Dispositif selon l'une quelconque des revendications 1 à 32, dans lequel ledit dispositif peut être porté.

34. Dispositif selon l'une quelconque des revendications 1 à 33, dans lequel ledit dispositif comprend une base stable et au moins une extension mobile.
